# EUROPEAN PATENT SPECIFICATION

(11) **EP 3 397 749 B1**
(45) Date of publication and mention of the grant of the patent: **26.01.2022**
(21) Application number: 16831697.4
(22) Date of filing: 30.12.2016
(51) Int. Cl.: C12N 1/22, C12N 5/00

(54) **LAYERED CELL CULTURE PARTICLES AND METHODS OF MAKING THEREOF**
GESCHICHTETE PARTIKEL FÜR ZELLKULTUR UND VERFAHREN ZUR HERSTELLUNG DAVON
PARTICULES STRATIFIÉES POUR CULTURE CELLULAIRE ET LEURS PROCÉDÉS DE FABRICATION

(30) Priority: 30.12.2015 US 201562272828 P
(43) Date of publication of application: 07.11.2018
(73) Proprietor: Life Technologies Corporation, Carlsbad, CA 92008 (US)
(72) Inventor: PHELPS, Mwita, Carlsbad, CA 92008 (US); GULDE, Paul, Carlsbad, CA 92008 (US); FIKE, Richard, Clarence, NY 14031 (US); REYNOLDS, Mary, Carlsbad, CA 92008 (US); HASSETT, Richard, Carlsbad, CA 92008 (US)
(74) Representative: Thermo Fisher Scientific- Life Sciences Solutions Group
(86) International application number: PCT/US2016/069564
(87) International publication number: WO 2017/117559

(56) References cited:
- EP-A2- 0 193 829
- WO-A1-01/45677
- WO-A1-2013/096858
- WO-A2-02/36735
- US-A- 3 089 824
- US-A- 3 241 520
- RICHARD FIKE ET AL: "Advanced Granulation Technology (AGTTM). An alternate format for serum-free, chemically-defined and protein-free cell culture media", CYTOTECHNOLOGY, KLUWER ACADEMIC PUBLISHERS, DO, vol. 36, no. 1-3, 1 July 2001 (2001-07-01) , pages 33-39, XP019236700, ISSN: 1573-0778, DOI: 10.1023/A:1014045104525
- SAURABH SRIVASTAVA ET AL: "Fluid Bed Technology: Overview and Parameters for Process Selection", INTERNATIONAL JOURNAL OF PHARMACEUTICAL SCIENCES AND DRUG RESEARCH : IJPSDR, vol. 2, no. 4, 1 October 2010 (2010-10-01) , pages 236-246, XP055292024, IN ISSN: 0975-248X cited in the application
- Anonymous: "Spray Granulation Agglomeration Coating Pelletizing Innovative Technologies for Granules and Pellets", , 17 October 2011 (2011-10-17), XP055355183, Retrieved from the Internet: URL:http://www.glatt.com/fileadmin/user_up load/content/pdf_downloads/AB_innovative_t echnologies_en_111017.pdf [retrieved on 2017-03-15]
- Anonymous: "Process & Plant engineering + innovative Process technology Functionalization oF granules and Pellets", , 16 March 2015 (2015-03-16), XP055355197, Retrieved from the Internet: URL:http://www.ingredientsnetwork.com/Glat t_BRO_PTFFF_116_Funktionalisierung-Granula te-Pellets_2016-03_EN_160315_web-file06950 3.pdf [retrieved on 2017-03-15]
- REINHART DAVID ET AL: "Benchmarking of commercially available CHO cell culture media for antibody production", APPLIED MICROBIOLOGY AND BIOTECHNOLOGY, SPRINGER, DE, vol. 99, no. 11, 7 April 2015 (2015-04-07) , pages 4645-4657, XP035502970, ISSN: 0175-7598, DOI: 10.1007/S00253-015-6514-4 [retrieved on 2015-04-07]
- Anonymous: "CHO CD EfficientFeed (TM) B AGT (TM) Nutrient Supplement Product Catalog No. Amount Storage Shelf Life*", , 1 January 2012 (2012-01-01), XP055355300, Retrieved from the Internet: URL:https://tools.thermofisher.com/content /sfs/manuals/CHO_CD_EfficientFeed_B_AGT_PI .pdf [retrieved on 2017-03-15]
- Anonymous: "Advanced Granulation Technology (TM) (AGT (TM) ) Media for Bioproduction", , 1 January 2002 (2002-01-01), XP055355302, Retrieved from the Internet: URL:https://tools.thermofisher.com/content /sfs/brochures/332_021646_AGT_Bro.pdf [retrieved on 2017-03-15]
- Anonymous: "L-Glutamine & GlutaMAX Supplement | Thermo Fisher Scientific - DK", , 2 October 2020 (2020-10-02), XP055736102, Retrieved from the Internet: URL:https://www.thermofisher.com/dk/en/hom e/life-science/cell-culture/mammalian-cell -culture/media-supplements/glutamax-media. html [retrieved on 2020-10-02]
- Anonymous: "Folic Acid and Tetrahydrofolates in Cell Culture | Sigma-Aldrich", , 2 October 2020 (2020-10-02), XP055736105, Retrieved from the Internet: URL:https://www.sigmaaldrich.com/life-scie nce/cell-culture/learning-center/media-exp ert/folic-acid.html [retrieved on 2020-10-02]
- SALAZAR ANDREW ET AL: "Amino acids in the cultivation of mammalian cells", AMINO ACIDS, SPRINGER VERLAG, AU, vol. 48, no. 5, 1 February 2016 (2016-02-01), pages 1161-1171, XP035889636, ISSN: 0939-4451, DOI: 10.1007/S00726-016-2181-8 [retrieved on 2016-02-01]
- ALISA SCHNELLBAECHER ET AL: "Vitamins in cell culture media: Stability and stabilization strategies", BIOTECHNOLOGY AND BIOENGINEERING, vol. 116, no. 6, 11 June 2019 (2019-06-11) , pages 1537-1555, XP055736111, US ISSN: 0006-3592, DOI: 10.1002/bit.26942

## Description

### CROSS-REFERENCE

This application claims the benefit under 35 U.S.C. § 119(e) of U.S. Provisional Application no. 62/272,828, filed 30 December, 2015.

### FIELD OF THE INVENTION

The present invention relates to dry cell culture media, feeds, supplements, or concentrates comprising layered particles useful in culturing cells and other such applications. The invention relates to processes for preparing such layered compositions and methods of making cell culture compositions that are stable, for e.g., thermally, photo-chemically and/or to gamma irradiation. T

### BRIEF SUMMARY OF THE INVENTION

The invention is directed in part to methods of making a media or a feed composition for culturing cells, comprising: i) subjecting a dry powder to suspension in a moving column of a gas in a fluid bed apparatus; ii) introducing at least one solvent using a sprayer into the dry powder of step (i) to form a layered base particle; and iii) drying the layered base particle; wherein each solvent in the plurality of solvents has one component; or the same components; or each solvent has different components; or each solvent has a mixture of components; wherein the components are segregated into reactive species and into sensitive species that are sprayed separately to give a layered granule; wherein the reactive species and the sensitive species are components that interact adversely with each other.

The invention is also directed in part to methods of making a media or a feed composition for culturing cells, comprising: i) subjecting a dry powder to suspension in a moving column of a gas in a fluid bed apparatus; ii) introducing a first solvent using a first sprayer onto the dry powder of step (i) to form a base granule; iii) introducing at least one second solvent using a second sprayer onto the base granule of step (ii) to form a layered base granule; iv) drying the layered base granule ; wherein each solvent in the plurality of solvents has one component; or the same components; or each solvent has different components; or each solvent has a mixture of components; wherein the components are segregated into reactive species and into sensitive species that are sprayed separately to give a layered granule; wherein the reactive species and the sensitive species are components that interact adversely with each other.

In accordance with the above first aspects of the invention, the dry powder may be a basal media powder, a complete media powder, a cell culture feed, a cell culture supplement, a cell culture media or feed concentrate or an amino acid mixture.

In a second aspect, the first sprayer may be selected from the group consisting of: a top sprayer, a bottom sprayer and a tangential sprayer. Alternately, the first or the second sprayer may be selected from the group consisting of: a top sprayer, a bottom sprayer and a tangential sprayer. In a preferred embodiment, the second sprayer may be a bottom sprayer.

In a third aspect, the second sprayer may spray a plurality of solvents on to the base granule to form a multilayered base granule or multilayered base particle respectively. Alternately, the second sprayer may spray the plurality of solvents sequentially, such that there are intermittent drying steps between each spraying step. And alternately, the second sprayer may spray the plurality of solvents sequentially without any intermittent drying steps between each spraying step.

In a fourth aspect, each solvent in the plurality of solvents may have one component; or, each solvent may have different components. For example, each solvent may have a mixture of components. In a further aspect, the solvent comprises a reactive species. In another aspect, the solvent comprises a sensitive species.

The components to be sprayed through the solvent are segregated into reactive species and into sensitive species.

The reactive species and the sensitive species are sprayed separately to give a layered granule. In a particular aspect, the reactive species may be selected from the group consisting of one or more trace elements, one or more reactive amino acids, one or more reactive amino acids, one or more metal salts, one or more acid soluble reactive groups, one or more alcohol soluble reactive groups and one or more pH modifier groups.

In another further aspect, the sensitive species may be selected from the group consisting of one or more polyamines, one or more vitamins, one or more growth factor and one or more labile amino acids.

In a sixth aspect, an inlet gas is used in the methods which may be at about temperature 20° C to 30° C. In a further aspect the inlet gas used in the method may be at about temperature 25° C.

In a seventh aspect, the vitamins that may be sensitive are selected from the group consisting of: vitamin B12, biotin, choline, folic acid, niacinamide, pyridoxine, riboflavin, thiamine, ascorbic acid and para-aminobenzoic acid (PABA).

In an eighth aspect, the drying step may be at about temperature 50° C to 60° C.

In a ninth aspect, the base particle or the base granule may be dried to a moisture content of about 0.5% to 10%.

In yet another further aspect, the solvent additionally comprises at least one antioxidant, or at least one neutral species, or both.

In a tenth aspect, the layered base particle or the layered base granule is obtained through any of the methods described above.

In a eleventh aspect, the methods are directed to producing a nutritive medium layered particle, a nutritive medium supplement layered particle, a nutritive medium subgroup layered particle, the method comprising making a layered media particle according to any of the methods described above and wherein at least one layer on the base particle or base granule comprises a reactive species and/or at least one layer on the base particle or base granule comprises a sensitive species. In a further aspect, the reactive species may be selected from the group consisting of one or more trace elements, one or more reactive amino acids, one or more reactive amino acids, one or more metal salts, one or more acid soluble reactive groups, one or more alcohol soluble reactive groups and one or more pH modifier groups. Alternately, the sensitive species may be selected from the group consisting of one or more polyamines, one or more vitamins, one or more growth factor and one or more labile amino acids. In some embodiments, the dry powder may be selected from the group consisting of vitamins, bulk inorganic salts and sugars.

Media or feed compositions made by the methods of the present invention may be used in methods directed to culturing a cell in a liquid reconstituted from the layered media made as described above, the method comprising: i) reconstituting the layered media particle in a suitable liquid or buffer; wherein said layered media particle may be a cell culture medium, feed, supplement or concentrate; and ii) culturing the cell may be the reconstituted liquid under conditions favorable for the growth of the cell. In a further aspect, the culturing of the cell may be used to produce increased amount of a polypeptide. The polypeptide may be a recombinant polypeptide. Alternately, the culturing increases product production, increasing cell growth, as compared to a culture with liquid media not made from the layered media particles.

Disclosed but not claimed is a kit comprising: i) a first container comprising a layered particle or granule according any one of the above claims; wherein said layered particle or granule may be a cell culture medium, feed, supplement or concentrate; and ii) instructions for using the layered particle or granule for cell culture.

Also disclosed is a system comprising: a liquid medium reconstituted from a layered media particles according to the methods described above, and a cell. The reconstituted liquid medium may be used to cultivate a cell that produces a recombinant polypeptide, a virus, a secreted protein, or to cultivate a cell in suspension or a cell that may be attached.

A fifteenth aspect provides methods of making a media or a feed composition for culturing cells as described above wherein amino acids are used and the amino acids are selected from one or more of the twenty amino acids, their salts or derivatives thereof. In a further aspect, the amino acids are selected from one or more of glycine, alanine, arginine, aspartic acid, glutamic acid, histidine, isoleucine, methionine, phenylalanine, proline, hydroxyproline, serine, threonine, tryptophan, valine, tyrosine, cysteine and lysine.

Layered particles or granules obtained through the methods described above may be used for culturing a cell. The cell may be a mammalian cell. or selected from the group consisting of CHO, BHK, HEK, 293, and VERO cells. The cell may be a plant cell, an animal cell, a eukaryotic cell, a eukaryotic cell, an algal cell, a fungal cell, and a bacterial cell.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1: Schematic of Process for Making Culture Media Layered Granule Formulations. Bottom spray coating was performed with a GPCG 1.1 with a 6" Wurster Insert, 2 Liter Granulator/Dryer/Coater. Bottom Spray Layering- Layer composition varies for each granulation.
Figure 1a: Schematic diagram of exemplary single layer, single ingredient cell culture media layered granules - Layered Granule Prototype A.
Figure 1b: Schematic diagram of exemplary multi-layer, single ingredient in each layer cell culture media layered granules - Layered Granule Prototype B.
Figure 2a: SEM images of cell culture media layered granules Prototype A (Single Layer, Single Ingredient).
Figure 2b: SEM images of cell culture media layered granules Prototype-B (Multi-Layered, Single Ingredient).
Figure 3a: Prototype A Layered Feed Cell Viability Assay.
Figure 3b: Prototype A Layered Feed Cell Growth Assay.
Figure 4: Exemplary Process for the Production of Layered Media/ Feed Granules for Cell Culture.
Figure 5a: Schematic diagram of Formulation 1 Cell Culture Media Layered Granules.
Figure 5b: Schematic diagram of Formulation 2 Cell Culture Media Layered Granules.
Figure 6a: Formulation 1 Particle size analysis of non-coated, non-layered granules (blue bars) and coated (layered) granules (red bars).
Figure 6b: Formulation 2 Particle size analysis of non-coated, non-layered granules (blue bars); and the coated (layered) granules (red bars).
Figure 7a: Formulation 1 Cumulative Particle Size (sieve) Analysis before and after bottom spray coating: Sieve Fraction analysis of the granulated base powder before layering step. Particle size analysis of non-coated, non-layered granules (blue squares) and coated, layered granules (red diamonds).
Figure 7b: Formulation 2 Cumulative Particle Size (sieve) Analysis before and after bottom spray coating: Sieve Fraction analysis of the granulated base powder before layering step.
Figure 8a: Formulation 1 SEM images of coated (layered) granules of formulation 1 (F1) at magnifications Left panel: Top View 100x; Right panel: Cross-Section 1500x.
Figure 8b: Formulation 2 SEM images of coated (layered) granules of formulation 2 (F2) at magnifications Left panel: Top View 100x; Right panel: Cross-Section 1500x.
Figure 9a: Formulations 1, 2, 3: Granulated Base Powders Particle Size Analysis. Cumulative Sieve Fraction analysis. Sieve analysis of base powder granulation was performed with 3-5 grams of granulated material using 3-inch sieves.
Figure 9b: Formulations 1, 2, 3: Granulated Base Powders Particle Size Analysis. Sieve analysis of base powder granulation was performed with 3-5 grams of granulated material using 3-inch sieves.
Fig 10a: Formulations 1, 2, 3: Milled Granulated Base Powders Particle Size Analysis. Cumulative Sieve Fraction analysis for the base powder that was milled post-granulation.
Fig 10b: Formulations 1, 2, 3: Milled Granulated Base Powders Particle Size Analysis Bar Graph. Sieve analysis of base powder (milled post- granulation) was performed with 95-105 grams of material using 8-inch sieves.
Figure 11: Radiation Induced Nutrient Degradation in Layered Granulations. As seen from the figures, layering strategies when applied to gamma irradiated dry format medium (dose range 20kGy-70kGy) improved the stability of sensitive and/or reactive components compared to that of un-irradiated (0kGy) control medium.

### DETAILED DESCRIPTION OF THE INVENTION

The invention relates to methods of producing a dry powder media composition comprising layered base particles or layered base granules. RICHARD FIKE ET AL, "Advanced Granulation Technology (AGTTM). An alternate format for serum-free, chemically-defined and protein-free cell culture media", CYTOTECHNOLOGY, KLUWER ACADEMIC PUBLISHERS, Vol. 36, No. 1-3, or WO0236735 disclose methods of making media and feed compositions for cell culture using spray drying of the components in a fluid bed apparatus. WO2013096858 discloses encapsulating microsuspensions for cell culture media components. There are several documents that disclose coating of particles using spray drying which do not relate to media or feeds for cell culture (see e.g. WO0145677, EP0193829, SAURABH SRIVASTAVA ET AL, "Fluid Bed Technology: Overview and Parameters for Process Selection", INTERNATIONAL JOURNAL OF PHARMACEUTICAL SCIENCES AND DRUG RESEARCH Vol. 2, No. 4, pages 236 - 246, US3241520, US3089824, or retrieved from the internet: http://www.ingredientsnetwork.eom/Glatt_BRO_PTFFF_116_Funktionalisierung-Granulate-Pellets_2016-03 _EN _160315 _web-file069503.pdf and http://www.glatt.com/fileadmin/user_upload/content/pdf_downloads/AB_innovative_technologies_en_11 1017.pdf,

Dry powder media when mentioned in this disclosure may refer to dry powder media (basal media or complete media), dry concentrated cell culture media, dry powder feeds or supplements, dry powder concentrated feeds or supplements, dry buffer powders, which may be used in combination, or in part, or by itself (when it may be a complete medium) to culture a cell. Generally, culturing refers to culturing a cell *in vitro.* A production method for making layered media or feed particles or granules will be described hereinafter.

Cell culture media, feed or supplement formulations may contain chemicals that become unstable over time (i.e., degrade, oxidize, etc.), or unstable due to adverse interactions between sensitive and reactive components in the formulation itself. For example, glucose may react with some amino acids or with polyamines to form undesirable Maillard reaction products that precipitate out when dissolved in solution, and such reactions may be further enhanced in the presence of one or more of the following: high-energy or ionizing radiations, thermal damage, light, environmental chemicals, long term storage, mechanical shaking, etc. Thus cell culture media must be stored or shipped at lower temperatures, which increases cost of goods and may be cumbersome. To overcome such challenges, a method is needed to protect cell culture media components that would otherwise interact adversely, and this may be achieved through segregating or grouping cell culture ingredients, for example, reactive components may be spatially separated from sensitive components through layering them in separate layers, and in some embodiments, by mixing the sensitive component layer and/or the reactive components with quenchers of free radicals or of reactive species; quenching components like anti-oxidants, and/or with neutral components (for example, see the Table in Example 3 below). A desirable solution to the above problem would therefore be to provide a cell culture medium or a feed (basal or complete) for supplementation in a cell culture system, such that the sensitive components in the formulation and/or the reactive components of the formulation are separated spatially from one another through layering. Optionally, inert components and/or quenchers such as anti-oxidants may be mixed with the sensitive components, and/or, the reactive components, or, the inert components and/or quenchers such as anti-oxidants may be layered in between the reactive and the sensitive layer. The application of such layered, powdered cell culture media may prolong the stability of the product, and/or increase stability, for e.g., to thermal, irradiation, or photochemical radiations.

To prepare a layered granule of a multi-component cell culture medium, feed or supplement can be challenging because complete media for example, comprises 80 to 100 components, each needed in precise amounts to maintain viable cell growth and maintain robust protein titers.

To prepare a layered granule a sprayer, for e.g., a bottom sprayer may be used, to spray a solvent onto an un-granulated base powder or a granulated or agglomerated base powder. A wide range of particle sizes can be layered. Exemplary granulated powders include but are not limited to those made by spray/wet granulation, fluid bed spray drying, (see Srivastava et al.; International Journal of Pharmaceutical Sciences and Drug Research 2010; 2(4): 236-246). Thus, a first layer may be formed on the granule. The solvent may contain one or more chemicals /components, or a microsuspension of one or more chemicals / components. In certain embodiments, different chemical layers of controlled thickness can be created on a granule. Binders need not to be used in the solvent or at any stage to form or to layer the granule. In other words, the formulation of a cell culture medium, feed or concentrate need not be altered due to the addition of binders or additional components for granule formation. The granule may be layered using components already present in the formulation. Instead, the formulation's components are segregated or group as described below.

Segregation of chemicals/ components and Layering: Chemicals / components in the layered granule may be segregated or grouped according to, for e.g., their chemical reactivity/ properties; their susceptibility to thermal damage or radiation damage, or photochemical damage, or according to their solvent solubility, etc.

For example, the core of the granule (also referred to as the base granule) may comprise one or more sensitive components (sometimes called inner core/layer). In other instances, the base granule may comprise a mixture of sensitive and anti-oxidant components. In yet other instances, the base granule may comprise a mixture of sensitive components, anti-oxidant and/or neutral components. For example, in a particular embodiment, the core of the granule may comprise the most sensitive component or a mixture of the most sensitive components, and, a mixture of one or more neutral (inert) components and/or one or more anti-oxidant components. In other instances, the base granule itself maybe layered such that the sensitive components, and/or neutral chemicals, and/or anti-oxidant components may be layered one by one, one on top of each other. For example, the most sensitive component may form the central core of the base granule, followed by the next sensitive component in the next layer, and so on, intermingled or followed by the neutral/ inert component(s) and/or, the anti-oxidant layer as the outermost layer of the base granule. Alternately, the neutral component may be at the core followed by sensitive components, or vice versa. The sensitive/ neutral / anti-oxidant layering of the granule may follow any order, according to the ingredients of the cell culture media, feed or concentrate and as determined by the skilled artisan based on the properties of cell culture media or feed components.

Next, the reactive chemicals may be applied over the sensitive component base granules (inner core or layer). In some examples, the reactive chemicals may be mixed together and applied as one layer over the base granule. This separation can provide some sort of spatial separation between the sensitive and the reactive chemicals within the same granule. In other instances, the reactive chemicals may be applied one by one, one layer over another, with the least reactive chemical being in contact with the (sensitive) base granule, and the most reactive chemical being spatially distant from the base granule (outermost layer). Alternately, an intervening, neutral/inert chemical layer may separate the inner, sensitive layer from the outer reactive layer/ layers of the granule.

In a preferred embodiment, AGT agglomerated cell culture media may be produced using a fluid bed granulation method. To a given base particle, an additional fluid bed coating technique may be applied, for example, using a bottom -spray layering method like the Wurster method. In an AGT cell culture formulation, the bottom spray method could allow chemical groups to be added in specific order to form controlled layers around bulk granulation components (i.e., called active layering) in order to separate/segregate reactive chemical components in the final AGT product. In some aspects, the coated particle can be modified for slow release of components, and in other aspects, the active layered AGT could provide protection of formulation components that are unstable to gamma irradiation, environmental degradation, degradation due to moisture, and so on. The reactive chemical components may be mixed with anti-oxidants to provide a protective effect. One or more active layers may be sprayed on an AGT particle to complete the formulation. Each layer may or may not comprise one or more antioxidants for additional stability. This method has potential to provide a terminally sterilized complete AGT medium (i.e., single-part, dry format) without the need for secondary processing conditions (e.g., spray drying, microencapsulation). Stability testing can be done after 1 week, 3 weeks, or more with storage of the layered particle media @ 37C, RT, 4°C, 0C, etc. Analytical testing against irradiation and/or environmental degradation, temperature variation, or weeks of stability was done by assaying for preservation of sensitive components such as vitamins, ethanolamine, etc. using routine assays.

Exemplary sensitive or less stable chemicals /components include but are not limited to, vitamins, polyamines, growth factors, hormones, etc. Exemplary reactive chemicals/ components include but are not limited to trace metals or salts, some reactive amino acids, some sugars (for e.g., glucose which may form adducts with other components including certain amino acids, polyamines), metal ions, etc. Exemplary neutral chemicals/ components include but are not limited to neutral amino acids, neutral sugars, etc.

### Definitions

The term "powder" or "dry powder" as used herein refers to media powders or powdered media compositions for cell culture that are present in dry form, whose gross appearance may be free flowing. The term "powder" includes agglomerated powders. The term "base powder" or "dry base powder" or "dry powder" may be used interchangeably, and as used herein generally refers to a starting dry powder composition before it may be layered. The term "base powder" or "dry base powder" or "dry powder" may be granulated by any means, for example, in a fluid-bed processor to produce a "base granule" or an "agglomerated granule" that can then be layered by further "layering" with solvents. But that does not mean that a layered particle (or granule) must start from a base granule. The layered particle can begin with a simple un-granulated powdered that may be layered using spraying techniques as well. For the sake of convenience, a granulated or agglomerated start particle before layering may be referred to as "a base granule". Correspondingly, a dry powder, un-granulated start particle may be sometimes referred to as "a base particle" before layering. It may not mean that any starting material may be completely free of dry powder or agglomerated granules. The starting material may sometimes have a mixture of dry powder and agglomerated granules before layering with a solvent. The dry or the agglomerated powder (as applicable) may be a basal media powder, a complete media powder, a cell culture feed, a cell culture supplement, a cell culture media or feed concentrate or an amino acid mixture.

The term "cell culture media compositions" or "powdered media composition" or "media powders" or "dry powder formulations" or "media formulations" may be used interchangeably and broadly include, *e.g.* a media, media supplement, media subgroup or buffer of the invention and may not be limited to, basal media, complete media, media feeds, media supplements, media additives, concentrated media, concentrated supplements and feeds, amino acids or groups of amino acids, short peptides, vitamins, buffers, salts, trace components, etc. These terms generally refer to components added during cell culture, and the skilled artisan would clearly understand when or how these terms are used.

The term media "component" or "ingredient" refers to any compound, whether of chemical or biological origin, that can be used in the cultivation of a cell, to maintain or promote the growth and proliferation of cells. The terms "component," "nutrient", "ingredient", "species", can be used interchangeably and are all meant to refer to such compounds. Typical ingredients that are used in cell culture media include amino acids, salts, metals ions, trace elements, polyamines, sugars, carbohydrates, lipids, nucleic acids, hormones, vitamins, fatty acids, buffer salts, proteins and the like. Other ingredients that promote or maintain cultivation of cells *ex vivo* can be selected by those of skill in the art, in accordance with the particular need.

Spraying: In certain embodiments, the "component" for layering may be sprayed on to the base particle, or the base granule. In certain embodiments, a singular "component" may be sprayed on to the base particle, or the base granule. In other embodiments, multiple "components" are sprayed. When multiple "components" are sprayed, they may be grouped together, for example, according to their reactivity, their solubility, their properties (for e.g., amino acid versus sugar), and sprayed together as a mixture. Alternately, the components may be sprayed one after another sequentially.

The term "solvent" refers to any liquid, such as water, any hydrant, any buffer, or an organic liquid like ethanol, etc. that can dissolve one or more "components" that needs to be layered. Layering may occur through the "spraying" of the liquid with the dissolved "component' or "mixture of components". The solvent may dissolve one or more "like" components; for e.g., ethanol may dissolve lipids, fatty acids, cholesterol, etc. or the solvent may dissolve one or more "dissimilar" components like "reactive components" and "anti-oxidants" together, probably because, the anti-oxidants or other such molecules/ components help stabilize or slow-down the degrading steps of the reactive species or groups. The solvent may be sprayed sequentially or all at once. When sprayed sequentially, each solvent sprayed may have the same composition, or may have different compositions. In certain instances, a plurality of solvents can be sprayed on to the same base particle or granule to form a multilayered base particle or granule. In certain instances, the multilayered base particle or granule spatially separate active or reactive species (or ingredients) from sensitive species. In one embodiment, the rate of the solvent introduction may be between about 1 gm/min up to about 30 gm/ min. In another embodiment, the rate of the solvent introduction may be about 5 gm/min.

The term "sprayer" refers to the equipment that sprays the liquid carrying one or more components onto to the base particle or base granule. Sprayer may be selected from the group consisting of: a top sprayer, a bottom sprayer and a tangential sprayer. One sprayer may be used for making a granulated particle (say, agglomerated particle), whereas a second sprayer, in one embodiment, a bottom sprayer may be used to layer the solvents and the components as desired and described above.

"Reactive species" refers to those components that are easily react with other components in the medium or feed, thereby producing undesirable products such as adducts, cross-linked products, precipitates etc. Reactive species may self-react or may be activated to react due to exposure to higher temperatures, to irradiation, to the presence of other chemicals or reactive species, etc. exemplary reactive species include but are not limited to, metal ions, sugars that form adducts, trace metal components, reactive amino acids, etc.

"Sensitive species" refers to those components that are easily destroyed due to their unwanted reaction, with more reactive species or groups from the medium or feed. Sensitive (or labile) species may self-react or may be activated to react with other components, especially reactive species, due to their exposure to higher temperatures, to irradiation, to the presence of other chemicals or reactive species, etc. Exemplary sensitive species include but are not limited to, vitamins, certain amino acids, polyamines such as ethanolamine, etc., biological components like growth factors etc. which are destroyed over time, or at higher temperature, or upon exposure to irradiation, or due to a combination of one or more of these factors.

The phrases "cell culture medium," "culture medium," and "medium formulation" (plural "media" in each case) refer to a nutritive solution that supports the cultivation and/or growth of cells; these phrases may be used interchangeably.

The term "combining" refers to the mixing or admixing of ingredients in a cell culture medium formulation. Combining can occur in liquid or powder form or with one or more powders and one or more liquids. In another example, two or more powdered components may be admixed and then agglomerated to produce a complex mixture such as media, media supplements, media subgroups or buffers.

The term "particle size" refers to the size (which may be a distribution) of the layered particles determined through a 'sieve' measurement. In an embodiment, the layered particle or base granule size maybe about 0.05mm to 7 mm. In a preferred embodiment, the layered particle or base granule size may be about 0.05 mm to about 0.5mm, about 0.05 mm to about 1mm, about 0.05 mm to about 2mm, about 0.05 mm to about 3mm, about 0.05 mm to about 4mm, about 0.05 mm to about 5mm, about 0.05 mm to about 6mm, about 0.05 mm to about 0.1mm, about 0.05 mm to about 0.2mm, about 0.05 mm to about 0.3mm, about 0.05 mm to about 0.4mm, about 0.1mm to about 1mm, about 0.1mm to about 2mm, about 0.1mm to about 3mm, about 0.1mm to about 4mm, about 0.1mm to about 5mm, about 0.1mm to about 6mm, about 0.5mm to about 1mm, about 0.5mm to about 2mm, about 0.5mm to about 3mm, about 0.5mm to about 4mm, about 0.5mm to about 5mm, about 0.5mm to about 6mm, about 0.5mm to about 7mm, about 1mm to about 2mm, about 1 mm to about 3mm, about 1 mm to about 4mm, about 1 mm to about 5mm, about 1 mm to about 6mm, about 1 mm to about 7mm. In yet another embodiment, the size of the layered particle or granule may be larger than about 0.5mm, a size larger than about 0.4 mm, larger than about 0.3 mm, larger than about 0.2 mm, larger than about 0.6 mm, larger than about 0.7 mm, larger than about 0.8 mm, larger than about 0.9mm, larger than about 1mm.

### EXAMPLES

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Example 1: Exemplary Process for making a Layered Cell Culture Media Granule - Prototypes A and B (using the Bottom Sprayer)

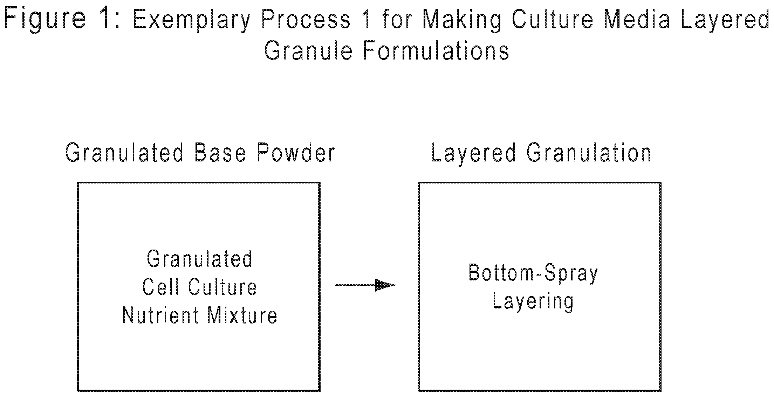

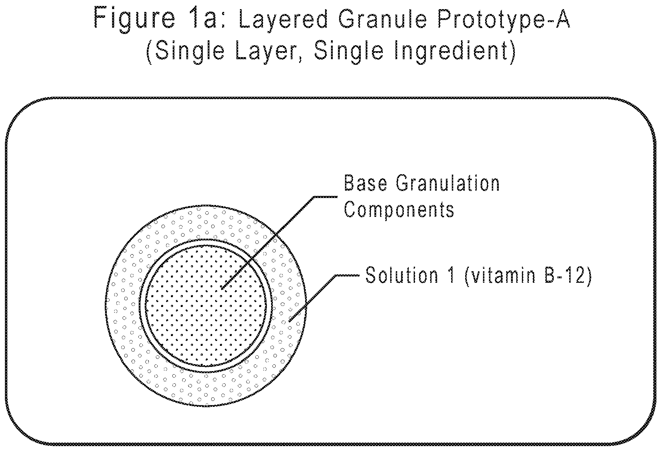

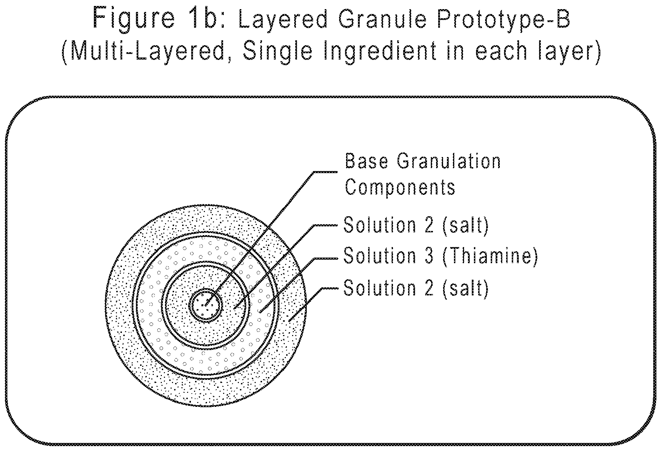

Granule formulations were designed and produced by layering methods by using a combination of a top sprayer for granulation and a bottom sprayer (for e.g., Wurster) for coating. In one example, bottom spray coating was performed with a GPCG 1.1 with a 6" Wurster Insert, 2 Liter Granulator/Dryer/Coater. For the bottom spray layering, the layer composition may vary for each granulation. For example, for prototypes A and B - (see Figures 1a, 1b; 2a, 2b; 3a, 3b). Granulated Base Medium for Prototypes A & B used SKU PL003021, Lot 1024MER1601. Base medium was produced by top-spray granulation.

Prototype A was layered as follows: Solution #1 (vitamin B-12), SKU PL003023, Lot 1101MER1601.

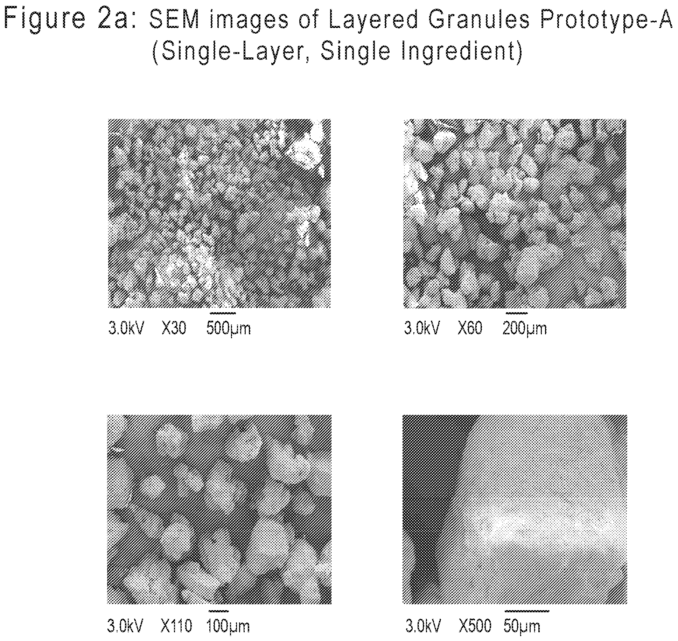

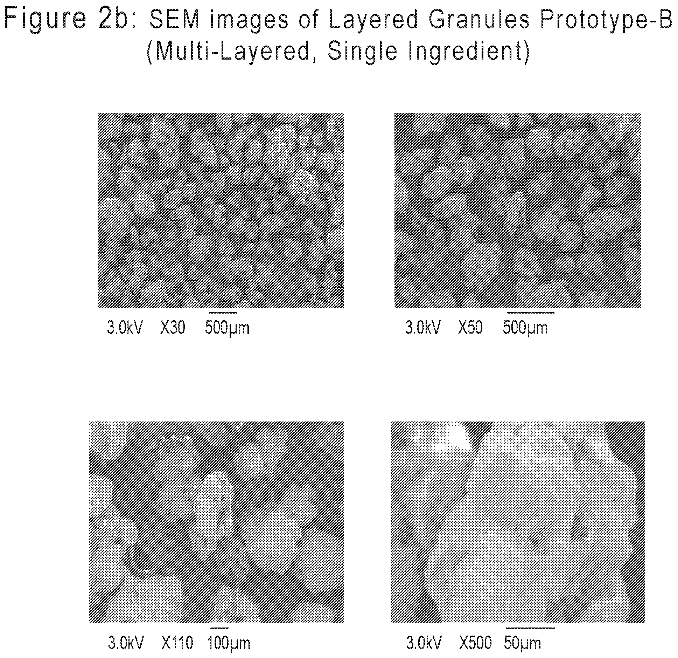

Apply Spray #1 (use ~95 ml per batch of 200X conc.) Prototype B was layered as follows: With Solution #2 (Salt solution NaCl,) SKU PL003024, Lot 1101MER1602. With Solution #3 (Thiamine HCl solution) SKU PL003029, Lot 1109MER1601. Apply Spray #2 (use ~38 ml per batch of 500X conc.) followed by Spray #3 (use ~38 ml per batch of 500X conc.). End with final application of Spray #2 (use ~38 ml per batch of 500X conc.). SEM images of Prototypes A and B are shown in Figures 2a and 2b. SEM of dried layered product shows Prototype A (bottom spray) with smoother surface compared to granulated base powder (top spray). SEM of Prototype B rough coated surface compared to granulated base powder (top spray) possibly because the outermost layer is a salt layer (NaCl).

### Example 2: Comparison of cell viability and growth in an assay of reconstituted layered feed media

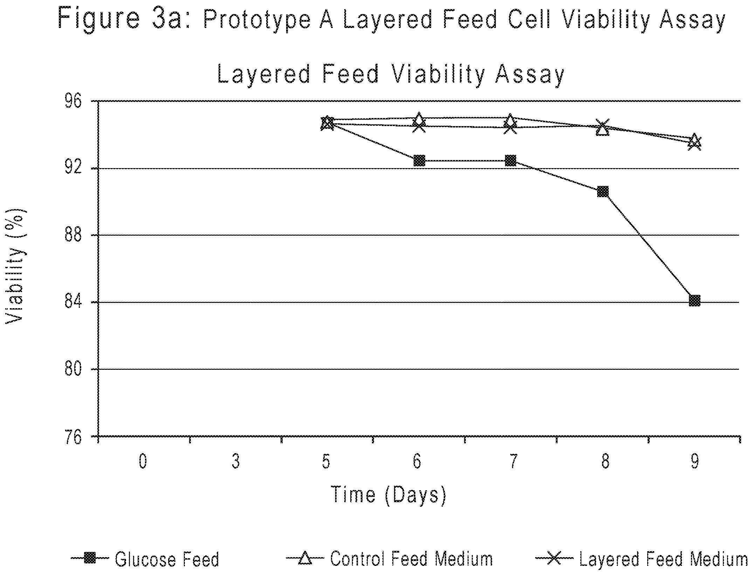

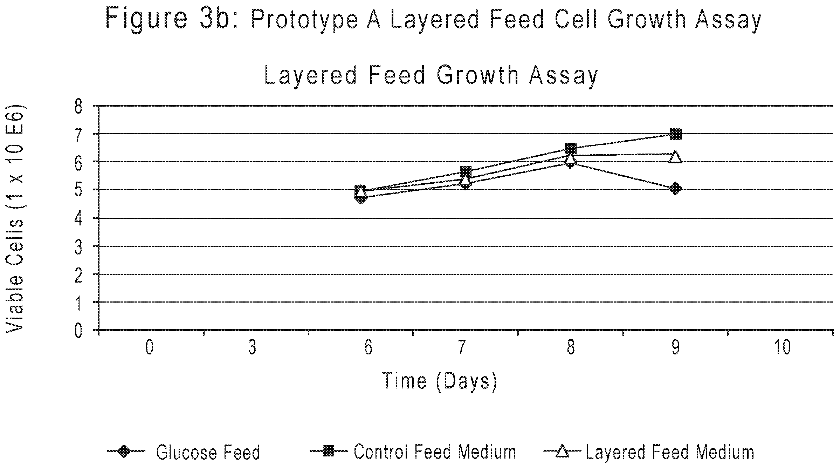

Comparison of cell viability and growth in an assay of reconstituted layered feed media (test= Prototype A tested) against positive control regular feed media (see Figures 3a and 3b). For this assay, DG44-IgG cell line was cultured in CD- CHO (medium) # 12490 with Efficient Feed B (supplement) #A2530 (positive control); while the test was reconstituted layered feed media (Prototype A); and negative control = media fed with glucose.

All cells were sub-passaged as follows: 3 subpassages for 3 days; then cells from each subpassage was split to 3 x 10 e5 viable cells/ml. At 4^{th} subpassage, after 3 days, daily cell counts were taken. All test and control feed media were reconstituted with liquid, then sterile filtered. As seen from Figures 3a and 3b, Prototype A showed high % viability comparable to cell culture supplemented with the unlayered, Positive Control Feed Medium. For cell growth, Prototype A showed viable cell counts of ~ 6.6 x 10 e6 cells /ml (growth), that is, only slightly lower numbers than the cell culture supplemented with the Positive Control Feed (~7.5 x 10 e6). For comparison, un-supplemented cell culture assay may be labeled as Glucose Feed (Negative Control).

### Example 3: Exemplary Process for the Production of Layered Media/ Feed Granules for Cell Culture

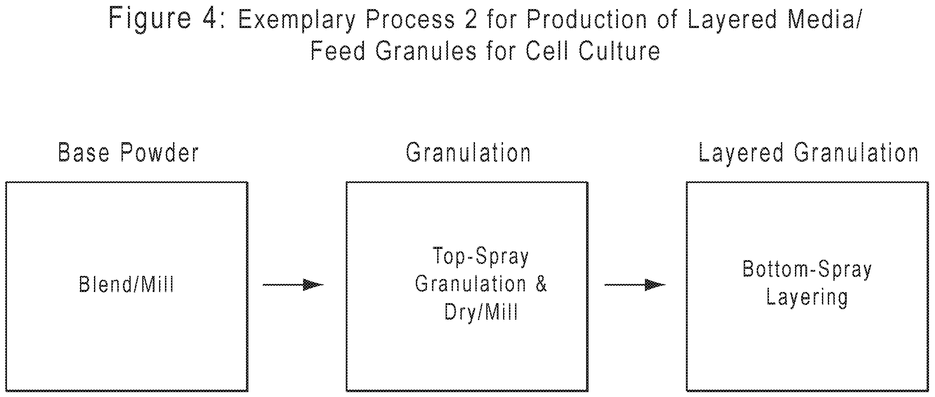

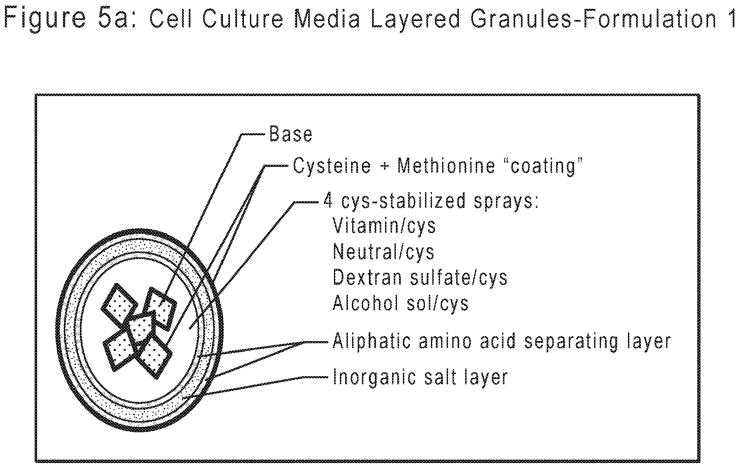

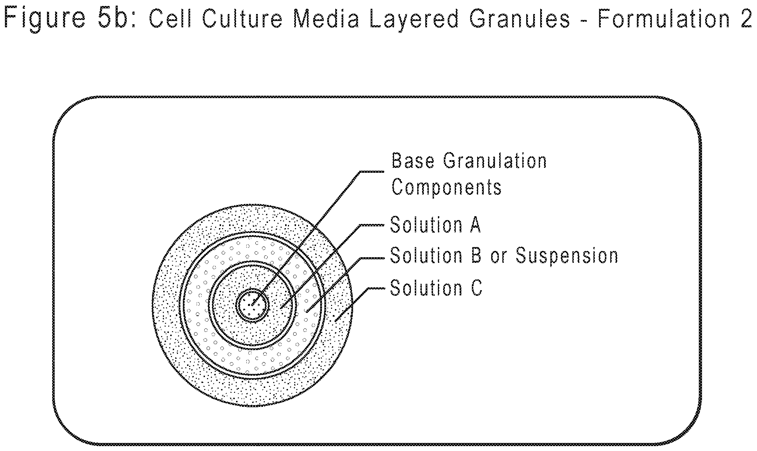

Granule formulations were designed and produced by layering methods, using a combination of a top sprayer for granulation and a bottom sprayer (Wurster) for coating. See Figures 4, 5a and 5b.

A base powder contains amino-acids, d-glucose, salts buffers and select vitamins (SKU 12681).

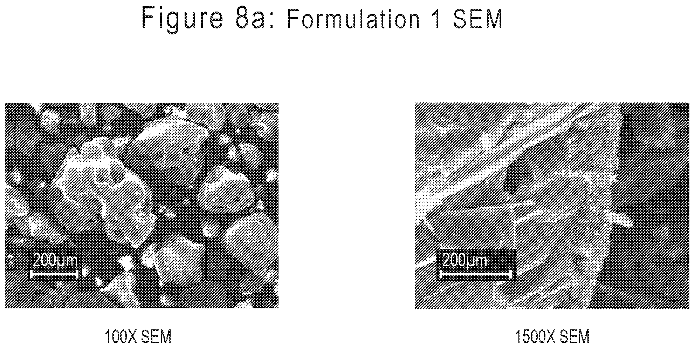

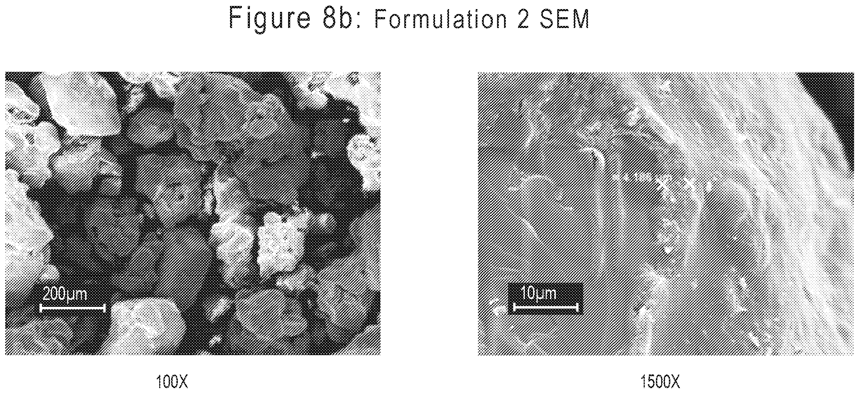

The powder was blended, for e.g., using a Blender (4qt), granulated using a solvent (for e.g., water) using a top-spray process (see Table 1) and with equipment including but not limited to, for e.g., Niro MP-1, Peristaltic Pump, Quadro Comil U5; then milled, for e.g., using a Fitzmill L1A. Here, the base powder was granulated before layering, but granulation of the base powder may not be a necessary step. Granulation may use top spray, bottom spray or tangential spray. Layering may be usually done using bottom spray layering and the Layer composition may vary for each granulation. See Table 1 below for top spray granulating parameters, and see Figures 4 - 11.

**Table 1: Exemplary Top Spray granulating parameters**

| **Formulation** | **Batch size (g)** | **Initial Granulation Parameters** | **7 min Granulation Parameters** | **20 min Granulation Parameters** | **30 min Granulation Parameters** |
|---|---|---|---|---|---|
| 1 | 1312 | Bed temp: 44°C | Bed temp: 30°C | Bed temp: 34.5°C | Bed temp: 39°C |
| | | Gas flow: 20 CMH | Gas flow: 75 CMH | Gas flow: 60 CMH | Gas flow: 60 CMH |
| | | Outlet T_{d}: 5°C | Outlet T_{d}: 13.5°C | Outlet T_{d}: 14.7°C | Outlet T_{d}: 10°C |
| | | Bed LOD: 0.7wt% | Bed LOD: 2.2wt% | Bed LOD: 2.2wt% | Bed LOD: 1.7wt% |
| 2 | 1437 | Bed temp: 44°C | Bed temp: 31°C | Bed temp: 34°C. | Bed temp: 36°C |
| | | Gas flow: 20 CMH | Gas flow: 75CMH | Gas flow: 60 CMH | Gas flow: 60 CMH |
| | | Outlet T_{d}: 3°C | Outlet T_{d}: 13.5°C | Outlet T_{d}: 11°C | Outlet T_{d}: 6°C |
| | | Bed LOD: 0.7wt% | Bed LOD: 2.8wt% | Bed LOD: 2.3wt% | Bed LOD: 1.9wt% |

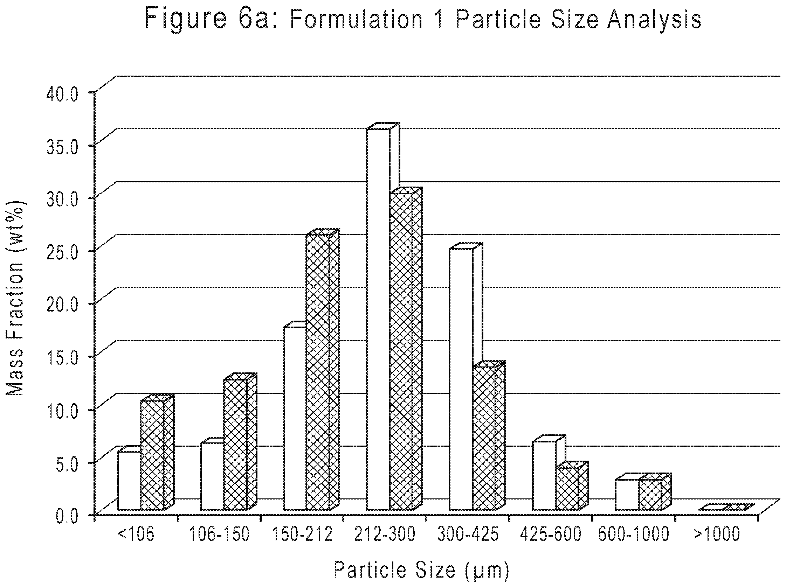

Granulated Base powders were subjected to particle size and SEM analysis (see Figures 6a and b; 7a and b; and 8a and b). Exemplary process parameters that may be used for making a granule are as follows; see Table 2:

| **Table 2: Process Parameters** | **Value** | **Unit** |
|---|---|---|
| Fluidization air Flow rate | 45 | CFM |
| Inlet Temperature (process) | 50 | °C |
| Inlet dew point | <5 | °C |
| Liquid spray rate | 5 +/- 0.5 | g/min |
| Atomization air pressure | 1.1 | bar |
| Bed Temperature | 35 +/- 2 | °C |
| Exhaust dew point | <10 | °C |

| **Product Parameter** | **Value** | **Unit** |
|---|---|---|
| Product Temperature Target | < 40 | °C |
| % final moisture | <2.5 | % |

In Table 2 (above), bottom spray coating was performed with a Niro MP-1 with a 6" modified bowl, a Schlick 970 nozzle and a 1.2 mm liquid tip (range 0.5-4.0 mm). The Nozzle was fixed at 4 cm above the base with a column air gap of 20 mm (range). There were 7 coatings for formulation 1 and 8 coatings for formulation 2. All coating runs were at least 20 minutes to ensure uniform coating. Between each coating, 5 mL of water was washed through the lines. At the end of the run, there was a 10 minute drying step with the atomizer off and the inlet temperature set to 40°C.

There were 7 coatings for formulation 1 and 8 coatings for formulation 2. A schematic diagram of each layer sprayed for Formulations 1 & 2 are shown in Figures 5a and 5b, and the description is also given below in Table 3.

**Table 3: Description of Formulation 1 & 2: Contents of the base granules and coatings that were sprayed.**

Descriptions are relative to Thermo Fisher catalog (catalog blend) CD OptiCHO AGT SKU# A11222-05.

| | **Formulation 1** | | **Formulation 2** | |
|---|---|---|---|---|
| | ***Description*** | ***Total wt%*** | ***Description*** | ***Total wt%*** |
| **Base** | • Remove cys, cystine (cys₂), met | 82% | • Remove cys, met | 94% |
| | • Remove aliphatic amino acids^{a} | | • Add post-granulation NaCl | |
| **Spray 1** | • Vitamin spray with glutathione, cys, met | 0.7% | • Vitamin spray with glutathione, cys, met | 0.7 % |
| | • 19% cys, 34% met (solids weight) | | • 26% cys, 39% met (solids weight) | |
| | • 11% w/v solids in water | | • 11% w/v solids in water | |
| **Spray 2** | • Neutral spray with dextran sulfate, cys, met | 0.6% | • Alcohol Solution | 0.01% |
| | | | • 0.11% w/v solids in 60% EtOH/water | |
| | • 3.1% cys, 5.7% met (solids weight) | | | |
| | • 8.8% w/v solids in water | | | |
| **Spray 3** | • Alcohol spray with cys, met | 0.01% | • Neutral spray with dextran sulfate, cys, met | 0.5% |
| | • 12% cys, 22% met (solids weight) | | | |
| | • 0.16% w/v solids in 60% EtOH/water | | • 8.8% w/v solids in water | |
| **Spray 4** | • Aliphatic amino acids | 3.5% | • Amines^{b} with cys and met | 0.7% |
| | • 4.9% w/v solids in water | | • 20% cys, 36% met (solids weight) | |
| | | | • 11% w/v solids in water | |
| **Spray 5** | • Iran chelate spray | 0.02 % | • Iron chelate spray | 0.02% |
| | • 0.31% w/v solids in water | | • 0.31% w/v solids in water | |
| **Spray 6** | • Trace element spray A | 0.01% | • Trace element spray A | 0.01% |
| | • 0.15% w/v solids in water | | • 0.15% w/v solids in water | |
| **Spray 7** | • Trace element spray B | 0.0002% | • Trace element spray B | 0.0002% |
| | • 5e-3% w/v solids in water | | • 5e-3% w/v solids in water | |
| **Spray 8** | • Aliphatic amino acids with cys and met | 3.9% | | -- |
| | • 3.7% cys, 6.7% met (solids weight) | | | |
| | • 5.5% w/v solids in water | | | |
| **Blend** | • Catalog Blend | 9.5% | • Pluronic F68 only | 3.9% |

**For Formulation 1:** Concept design #1: to achieve sensitive group protection using bottom spray layering (coating) method (Multi-layered, Multi-Ingredients) and consists of the following layers:
Base granulation ingredients (e.g., D-glucose, salts, amino acids, buffers and some vitamins) coated with a first layer of antioxidants ("Core").Then coated with a second layer of nutrients that have anti-oxidant properties. Followed by a third layer that contains sensitive nutrients (i.e., concentration decreases as a result of gamma irradiation) mixed with anti-oxidant nutrients. Then a fourth layer may be applied as a barrier to separate sensitive ingredients from the remaining 3 layers which include: a salt layer, additional separation barrier layer, an outer antioxidant layer ("Shell").

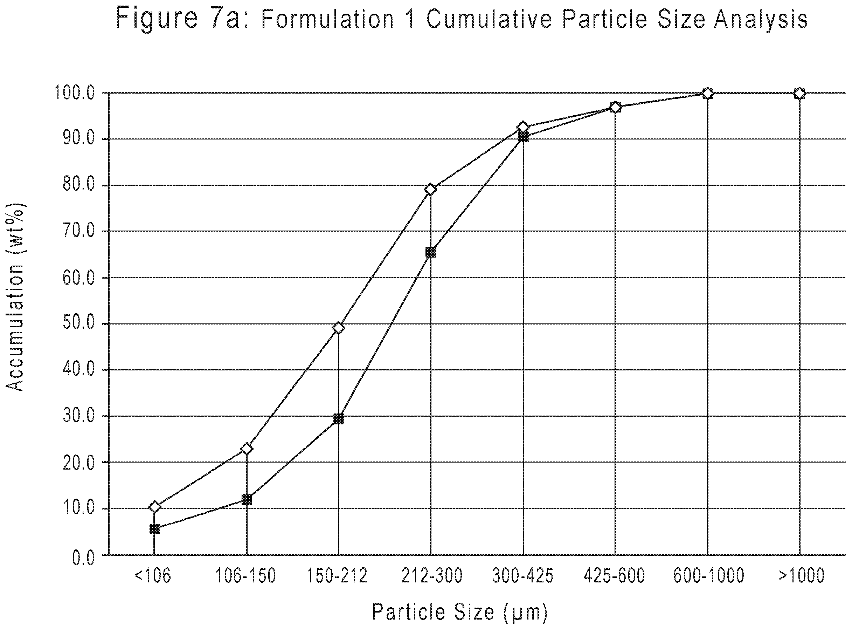

Formulation 1 included 8 coats with a total coating thickness of about 10 µm, and D50 = 180 µm. Sieve analysis before and after bottom spray coating was performed (see Figures 6a and 7a). The coated granules have a smaller average size than the starting uncoated granules due to granule attrition during the process. Sieve Analysis used for example, the Retsch sieve shaker, but any shaker can be used. Details: 8" sieves, amplitude 6, pulsed, 6 minutes duration.

An exemplary layered AGT Prototype or "Onionskin" layered AGT was produced using bottom spray method. The layered AGT prototype was made where reactive groups were segregated/ layered. The method was scalable process up to 1-2 kg. Layer formation was identified and confirmed by analytical methods. An exemplary prototype product could consist of the following three layers: Bulk granulation components, Solution A= Vitamins, Solution B (or Suspension)^{∗} = anti-oxidant amino acid layer (for example, Cysteine & Methionine), Solution C = Trace Elements (or other oxygen reactive components). ^{∗}Note- Cysteine can be formulated as a solution in one granulation. Cysteine has the potential to form cystine (dimerization) in solution. One approach to avoid the formation of cystine may be to formulate cysteine as micro-suspension in a second granulation.

**For Formulation 2:** Concept design #2: to achieve reactive group segregation using bottom spray layering (coating) method (Multi-layered, Multi-Ingredients) and consists of the following layers:

Base granulation ingredients (e.g., d-glucose, salts, amino acids, buffers and some vitamins). Solution A contains sensitive nutrients (i.e., concentration decreases as a result of gamma irradiation). Solution B contains nutrients that have anti-oxidant properties. Solution C contains nutrients that may be reactive toward base granulated components.

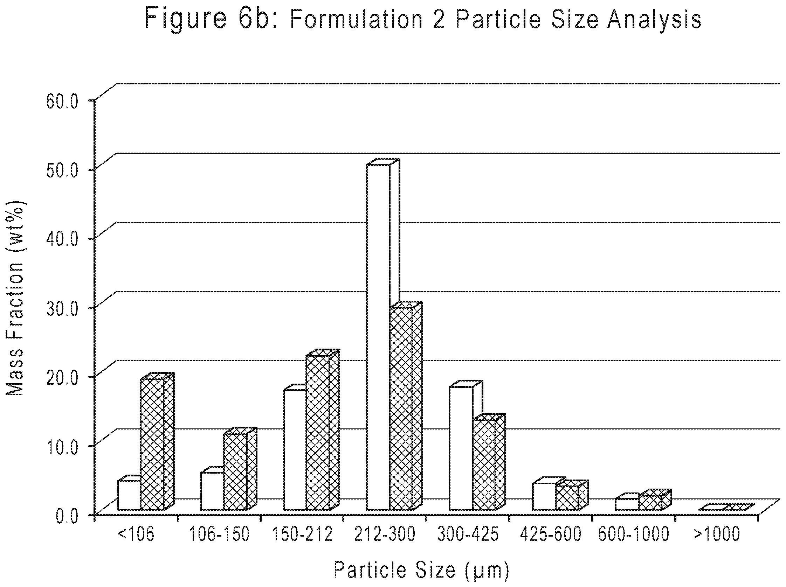

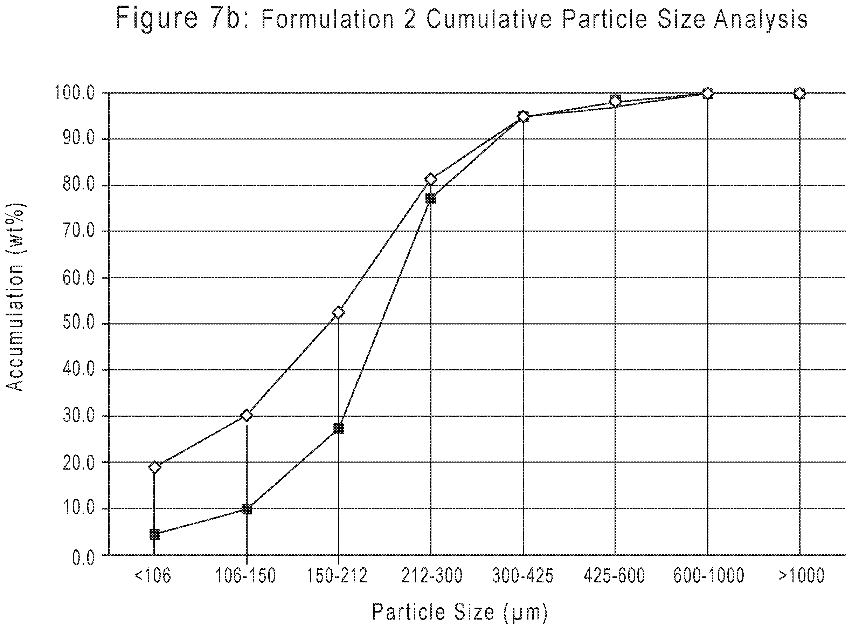

Formulation 2 included 7 coats with a total coating thickness of about 5 µm and D50 = 180 µm. "Onionskin" layered (thin layers). Sieve analysis before and after bottom spray coating was performed (see Figures 6b and 7b).

An exemplary layered media, Formulation 2 was prepared as described: Bulk granulation components- list of media chemicals for granulation step; 1st inner layer; (Vitamins w/Antioxidants) = for example, Vitamins + cysteine- HCl- H2O + methionine= 2nd layer; (Polyamines w/Antioxidants)^{∗} = for example, cysteine & methionine + polyamines + ethanolamine= 3^{rd} layer; Salts #1 = (Iron Chelate Solution), for example, EDTA + Ferrous Sulfate 7H2O= 4^{th} layer; Salts #2 (Trace Element Solution A (0116031)) = 5th layer ; Salts #3 (Trace Element Solution B (0116026))= 6th layer.

**For Formulation 3:** An exemplary layered media, Formulation 3 (F3) was prepared as described herein: "Core-Shell" layered AGT produced using bottom spray method ("Core-Shell" layering). Reduction to practice of micro-suspension layering method(s) (i.e. scalable process up to 1-2 kg) Formulation 3 (F3) is described herein: Bulk granulation components, 1st inner layer, Coating #2 (Vitamins w/Antioxidants) = Vitamins + cysteine- HCl- H2O + methionine=2nd layer; (Antioxidants microsuspension)^{∗} = (cysteine & methionine micronized/suspended in Pluronic/water carrier solution or PEG/water carrier solution) = 3^{rd} layer; Salts #1 = (Iron Chelate Solution)= EDTA + Ferrous Sulfate, 7H2O = 4^{th} layer; Salts #2 (Trace Element Solution A (0116031)) = 5th layer ; Salts #3 (Trace Element Solution B (0116026)) = 6th layer. Or layers 4, 5 and 6 may be combined.

^{∗}Note: Cysteine has the potential to form cystine (dimerization) in solution. One approach to avoid the formation of cystine may be to formulate cysteine as micro-suspension. Test the feasibility of using pluronic or PEG as solvents for micro-suspensions since these polymers are compatible with water soluble and water insoluble compounds.

Formulation Processes for Bottom-Spray Solutions: "Onionskin" layered AGT produced using bottom spray method. Steps for preparing Formulation 2 (F2) or Formulation 3 (F3) is described herein. Bulk granulation components (inner core). ^{∗}Note- Polyamines w/Antioxidants can be formulated as a solution, see below. Step 1. A solution of polyamines (pH range= 6.4-6.6) has been pre-formulated, sterile filtered (0.2um). Step 2. Add Antioxidants in the polyamine solution prepared in step 1, stir the solution (magnetic stir plate) until dissolved (a clear solution forms). Step 3. Measure & record the pH of the clear solution (pH range 2.0- 3.0). Step 4. Adjust the solution pH to 6.5 - 7.5 using 5N NaOH. The solution should remain clear and can be used to produce bottom-spray layered granulation.

**Physical Properties of Formulations 1 & 2**

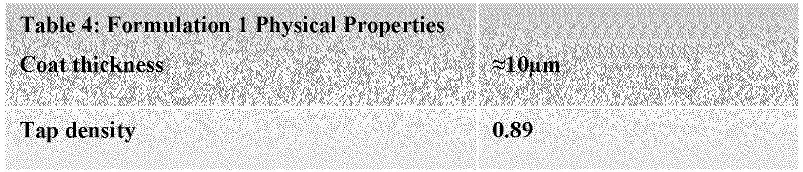

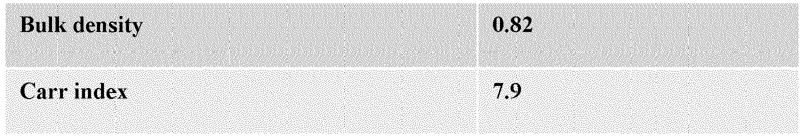

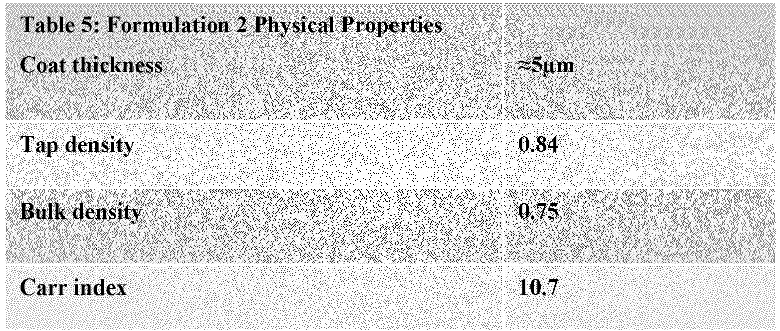

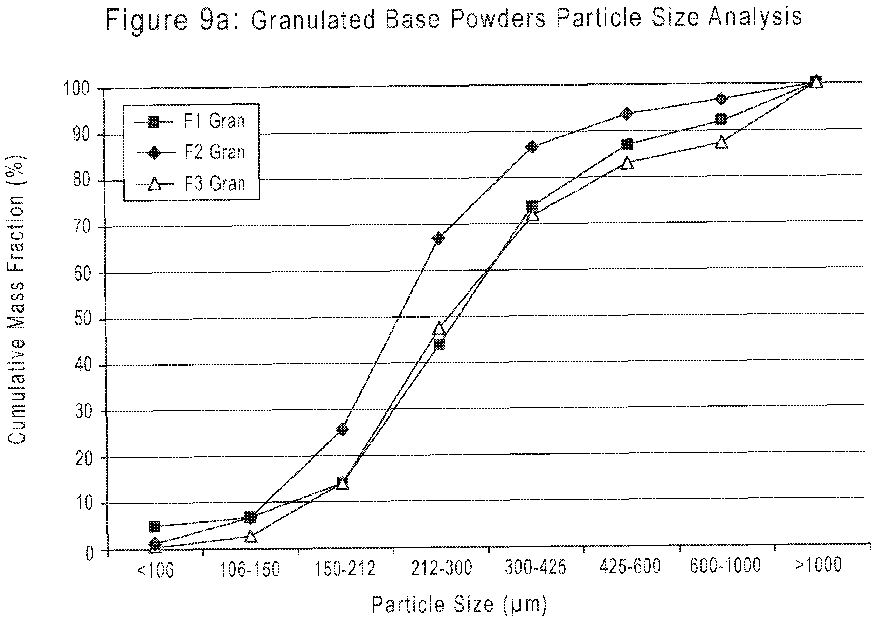

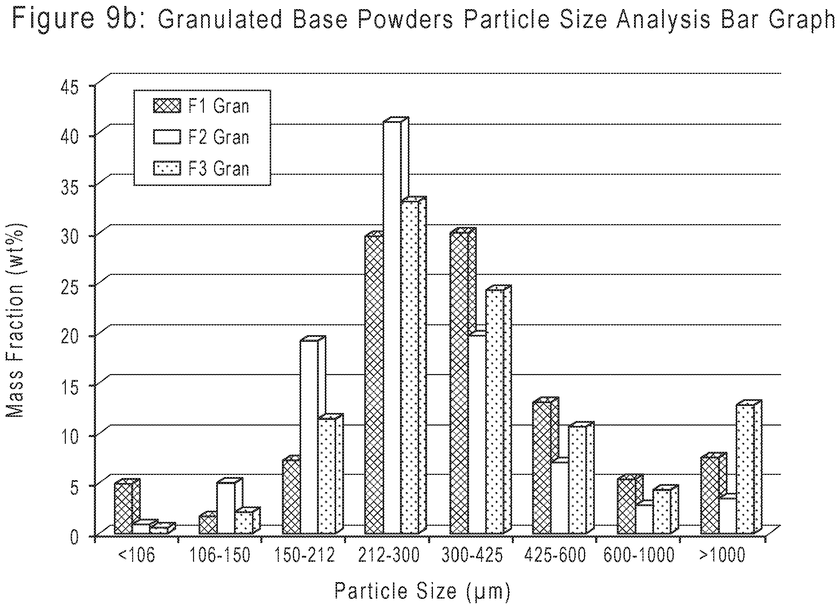

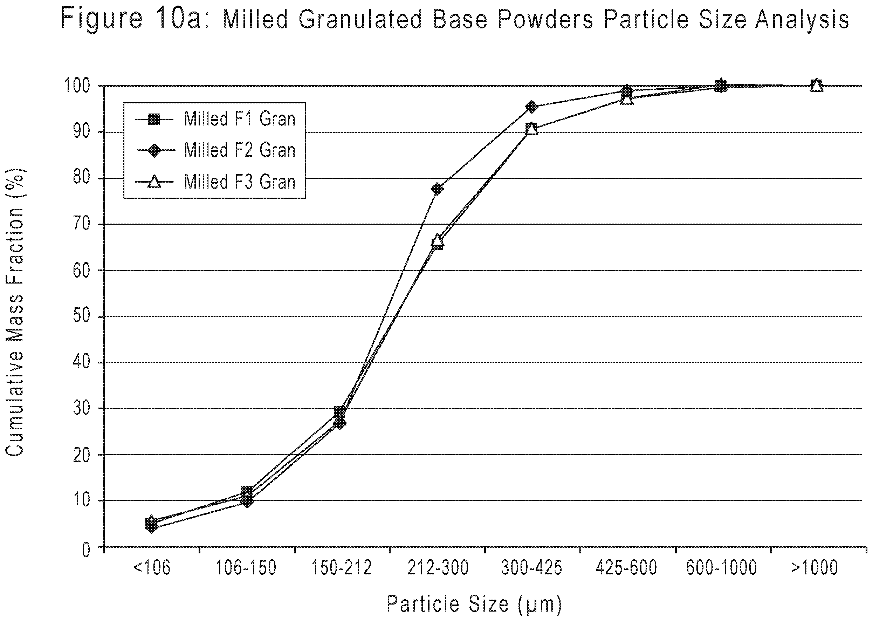

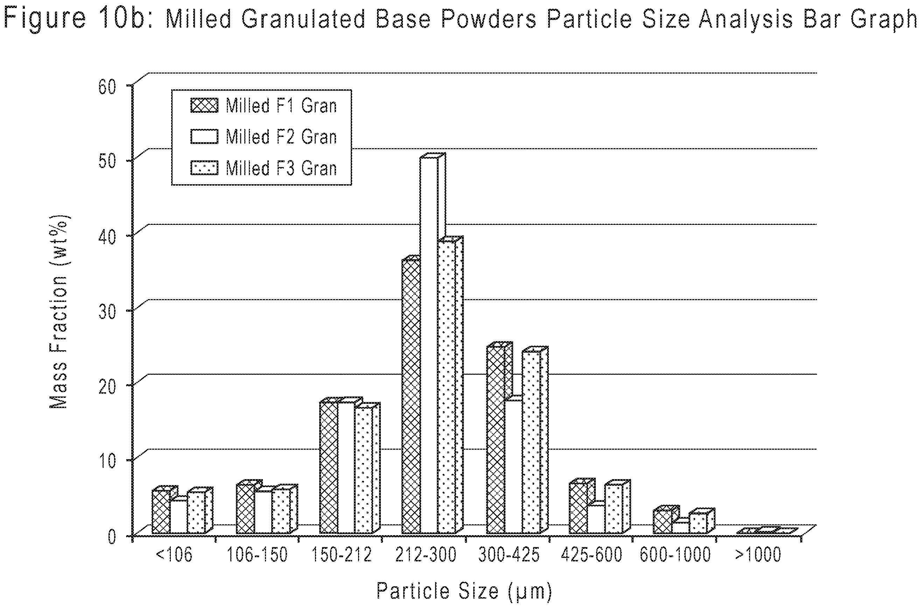

For comparison, the base granulated particle size (Figures 9a and b) and milled base granulated particle size (Figures 10a and b) were also performed.

### Example 4: Analytical testing of Formulation 1 & 2 before and after gamma radiation

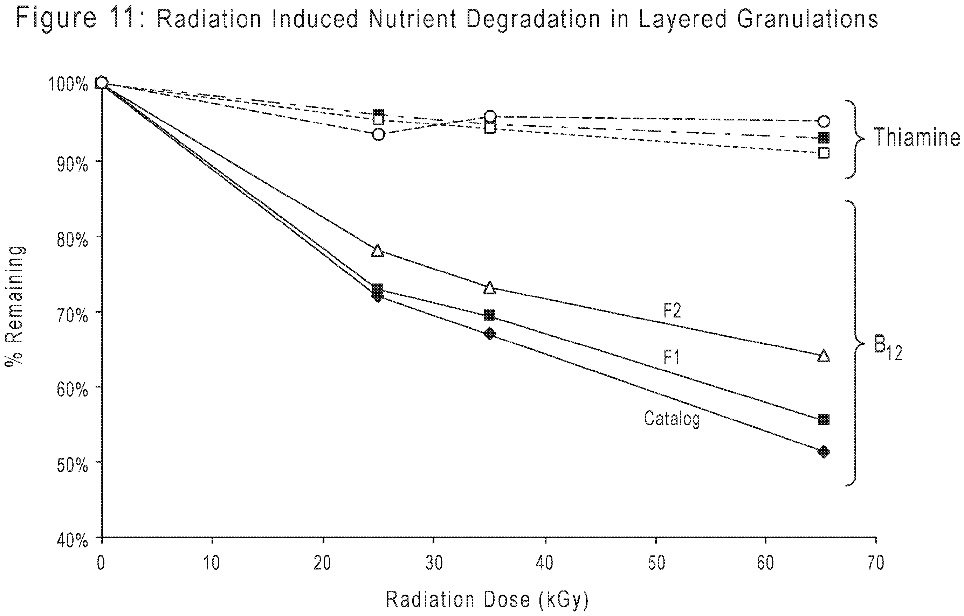

The dry, layered cell culture media (formulations 1 and 2) prepared by methods described above were tested further for gamma irradiation stability. The goal of this experiment was to determine if a radiation-tolerant media could be prepared by changing the process conditions only, here, layering media/feed components (i.e., without changes to the cell culture medium formulation). The layered media was treated with gamma irradiation in the dose range of about 20kGy to about 70 kGy as shown in the Table below. The gamma-irradiation tolerance of the resultant layered granulated media was analyzed by measuring the concentration of sensitive chemical markers (for e.g., vitamins like thiamine, vitamin B-12, etc.) before and after gamma radiation. For each formulation (F1 &F2) and gamma radiation condition, two 1-L media batches were prepared using MilliQ water. Each sample was also tested (by HPLC) for tryptophan, niacinamide, folic acid, thiamine, and vitamin B12. Dose column lists average dosage of gamma radiation that the formulation was exposed to for this study. The results are presented below in Table 6 and in Figure 11. In addition, physical characterization of the resultant granules was done (Tables 4 and 5). It was found that the multicomponent cell culture media prepared using bottom spray layering techniques gives excellent uniformity of content. It was also noted that controlled placement of each layer around a base granulated powder provided a method to physically separate reactive and sensitive chemicals within the same formulation. Grouped layers further improved chemical stability during exposure to conditions that cause thermal reactivity or degradation, photochemical reactivity or degradation, radiation induced reactivity or degradation.

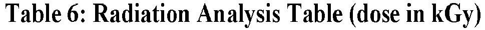

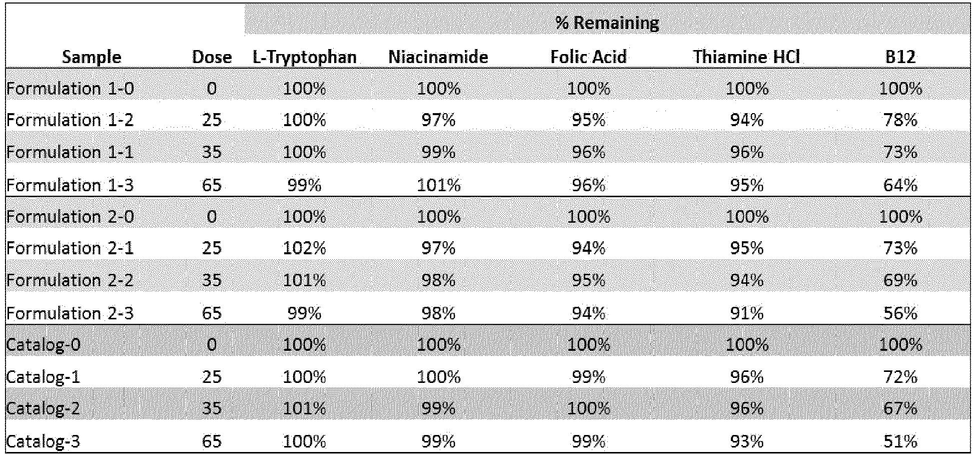

Summary of Table 6 and other Assays for Formulations 1 & 2: Thiamine degraded up to 9% in a dose-dependent fashion and vitamin B12 degraded as much as 49% in a dose-dependent fashion. The degradation of thiamine and vitamin B12 are shown in Figure 11. Vitamin B12 degradation at 60 - 70 kGy was reduced by 18% and 26% relative to the catalog control for formulations 1 and 2, respectively (Protection of gamma radiation sensitive nutrients in Layered granulations). Gamma Radiation Treatment-The catalog CD OptiCHO AGT control, formulation 1, and formulation 2 were shipped for example to Steris for gamma radiation treatment. All samples were stored at 5°C and were shipped and irradiated at dry ice temperature (-78°C). Each formulation was divided into four batches and kept either (a) at 5°C, or (b) irradiated with a total dose of (b) 20 - 30 kGy, (c) 30 - 40 kGy, or (d) 60 - 70 kGy.

Tryptophan and niacinamide assay levels were within 3% of the non-irradiated media for all radiation levels tested. Folic acid content was within 6% of the non- irradiated media for all radiation doses. Gamma Radiation Treatment-The catalog CD OptiCHO AGT control, formulation 1, and formulation 2 were shipped for example, to Steris, for gamma radiation treatment. All samples were stored at 5°C and was shipped and irradiated in dry ice temperature (-78°C). Each formulation was divided into four batches and either kept (a) at 5°C, or irradiated with a total dose of (b) 20 - 30 kGy, (c) 30 - 40 kGy, or (d) 60 - 70 kGy.

The processes described above are applicable for cultivation of any cell type: mammalian, bacterial, insect, fungal, etc., that derive their nutrient support from the reconstituted dry-format, layered, cell culture media described throughout this specification. As described in some embodiments above, also encompassed within the invention's scope is the application of a layer composed of ingredients formulated as a microsuspension, or as a coating for cell culture granulated media. The large number of permutations in which cell culture media ingredients can be grouped, separated or otherwise organized within layers around any powdered cell culture media/ feed, or granulated cell culture media/ feed makes this methodology useful for a wide variety of applications, such as, to increase thermal stability, radiation tolerance, modified dissolution profiles, etc., as will be readily appreciated by one of skill in the art.

## Claims

1. A method of making a media or a feed composition for culturing cells, comprising:
i) subjecting a dry powder to suspension in a moving column of a gas in a fluid bed apparatus;
ii) introducing at least one solvent using a sprayer into the dry powder of step (i) to form a layered base particle;
iii) drying the layered base particle;
wherein each solvent in the plurality of solvents has one component; each solvent has different components; or each solvent has a mixture of components;
wherein the components are segregated into reactive species and into sensitive species that are sprayed separately to give a layered granule;
wherein the reactive species and the sensitive species are components that interact adversely with each other.

2. A method of making a media or a feed composition for culturing cells, comprising:
i) subjecting a dry powder to suspension in a moving column of a gas in a fluid bed apparatus;
ii) introducing a first solvent using a first sprayer onto the dry powder of step (i) to form a base granule;
iii) introducing at least one second solvent using a second sprayer onto the base granule of step (ii) to form a layered base granule;
iv) drying the layered base granule;
wherein each solvent in the plurality of solvents has one component; each solvent has different components; or each solvent has a mixture of components;
wherein the components are segregated into reactive species and into sensitive species that are sprayed separately to give a layered granule;
wherein the reactive species and the sensitive species are components that interact adversely with each other.

3. The method of making the media or the feed composition for culturing cells of claim 1 or 2, wherein the dry powder is a basal media powder, a complete media powder, a cell culture feed, a cell culture supplement, a cell culture media or feed concentrate or an amino acid mixture, or wherein the dry powder is selected from the group consisting of vitamins, bulk inorganic salts and sugars.

4. The method of making the media or the feed composition for culturing cells of claim 1, wherein the first sprayer is selected from the group consisting of: a top sprayer, a bottom sprayer and a tangential sprayer; and/or further comprising a second sprayer that sprays a plurality of solvents onto the base particle to form a multilayered base particle.

5. The method of making the media or the feed composition for culturing cells of claim 2 or 3, wherein the first or the second sprayer is selected from the group consisting of: a top sprayer, a bottom sprayer and a tangential sprayer; and/or
wherein the second sprayer is a bottom sprayer; and/or
wherein the second sprayer sprays a plurality of solvents onto the base granule to form a multilayered base granule.

6. The method of making the media or the feed composition for culturing cells of any of the preceding claims, wherein the second sprayer sprays the plurality of solvents sequentially, with intermittent drying steps between each spraying step; and/or
wherein the second sprayer sprays the plurality of solvents sequentially, without any intermittent drying steps between each spraying step.

7. The method of any of the preceding claims, wherein said reactive species is selected from the group consisting of one or more trace elements, one or more reactive amino acids, one or more reactive amino acids, one or more metal salts, one or more acid soluble reactive groups, one or more alcohol soluble reactive groups and one or more pH modifier groups.

8. The method of any of the preceding claims, wherein said sensitive species is selected from the group consisting of one or more polyamines, one or more vitamins, one or more growth factor and one or more labile amino acids; optionally wherein the one or more vitamins are selected from the group consisting of vitamin B12, biotin, choline, folic acid, niacinamide, pyridoxine, riboflavin, thiamine, ascorbic acid and para-aminobenzoic acid (PABA).

9. The method of making the media or the feed composition for culturing cells of any of the preceding claims, wherein an inlet gas used in the method is at about temperature 20°C to 30°C, optionally at about temperature 25°C.

10. The method of making the media or the feed composition for culturing cells of any of the preceding claims, wherein the drying step is at about temperature 50°C to 60°C.

11. The method of making the media or the feed composition for culturing cells of any of the preceding claims, wherein the base particle or the base granule is dried to a moisture content of about 0.5% to 10%.

12. The method of making the media or the feed composition for culturing cells of any of the preceding claims, wherein the solvent comprises a reactive species.

13. The method of making the media or the feed composition for culturing cells of any of the preceding claims, wherein the solvent additionally comprises at least one antioxidant.

14. The method of making the media or the feed composition for culturing cells of any of the preceding claims, wherein inert components and/or quenchers such as anti-oxidants are mixed with the sensitive components, and/or, the reactive components, or, the inert components and/or quenchers such as anti-oxidants are layered in between the reactive and the sensitive layer.

15. The method of making the media or the feed composition for culturing cells of any of the preceding claims, wherein the components in the layered granule are segregated or grouped according to their chemical reactivity/properties; their susceptibility to thermal damage or radiation damage, or photochemical damage, or according to their solvent solubility.

## Patentansprüche

1. Verfahren zum Herstellen von Medien oder einer Feed-Zusammensetzung zum Kultivieren von Zellen, Folgendes umfassend:
i) Unterziehen eines trockenen Pulvers einer Suspension in einer sich bewegenden Säule eines Gases in einer Wirbelschichteinrichtung;
ii) Einbringen von wenigstens einem Lösungsmittel unter Verwendung eines Zerstäubers in das trockene Pulver von Schritt (i), um ein geschichtetes Basisteilchen auszubilden;
iii) Trocknen des geschichteten Basisteilchens;
wobei jedes Lösungsmittel der mehreren Lösungsmittel eine Komponente aufweist; jedes Lösungsmittel unterschiedliche Komponenten aufweist; oder jedes Lösungsmittel eine Mischung von Komponenten aufweist;
wobei die Komponenten in reaktive Spezies und in empfindliche Spezies segregiert werden, die getrennt zerstäubt werden, um ein geschichtetes Granulat zu ergeben;
wobei die reaktive Spezies und die empfindliche Spezies Komponenten sind, die nachteilig miteinander wechselwirken.

2. Verfahren zum Herstellen von Medien oder einer Feed-Zusammensetzung zum Kultivieren von Zellen, Folgendes umfassend:
i) Unterziehen eines trockenen Pulvers einer Suspension in einer sich bewegenden Säule eines Gases in einer Wirbelschichteinrichtung;
ii) Einbringen eines ersten Lösungsmittels unter Verwendung eines ersten Zerstäubers auf das trockene Pulver von Schritt (i), um ein Basisgranulat auszubilden;
iii) Einbringen von wenigstens einem zweiten Lösungsmittel unter Verwendung eines zweiten Zerstäubers auf das Basisgranulat von Schritt (ii), um ein geschichtetes Basisgranulat auszubilden;
iv) Trocknen des geschichteten Basisgranulats;
wobei jedes Lösungsmittel der mehreren Lösungsmittel eine Komponente aufweist; jedes Lösungsmittel unterschiedliche Komponenten aufweist; oder jedes Lösungsmittel eine Mischung von Komponenten aufweist;
wobei die Komponenten in reaktive Spezies und in empfindliche Spezies segregiert werden, die getrennt zerstäubt werden, um ein geschichtetes Granulat zu ergeben;
wobei die reaktive Spezies und die empfindliche Spezies Komponenten sind, die nachteilig miteinander wechselwirken.

3. Verfahren zum Herstellen der Medien oder der Feed-Zusammensetzung zum Kultivieren von Zellen nach Anspruch 1 oder 2, wobei das trockene Pulver ein Basalmedienpulver, ein vollständiges Medienpulver, ein Zellkultur-Feed, eine Zellkulturergänzung, Zellkulturmedien oder Feed-Konzentrat oder eine Aminosäuremischung ist, oder wobei das trockene Pulver aus der Gruppe ausgewählt ist, die aus Vitaminen, anorganischem Salz-Schüttgut und Zuckern besteht.

4. Verfahren zum Herstellen der Medien oder der Feed-Zusammensetzung zum Kultivieren von Zellen nach Anspruch 1, wobei der erste Zerstäuber aus der Gruppe ausgewählt ist, die aus Folgendem besteht: einem oberen Zerstäuber, einem unteren Zerstäuber und einem tangentialen Zerstäuber; und/oder ferner umfassend einen zweiten Zerstäuber, der mehrere Lösungsmittel auf das Basisteilchen zerstäubt, um ein mehrschichtiges Basisteilchen auszubilden.

5. Verfahren zum Herstellen der Medien oder der Feed-Zusammensetzung zum Kultivieren von Zellen nach Anspruch 2 oder 3, wobei der erste oder der zweite Zerstäuber aus der Gruppe ausgewählt ist, die aus Folgendem besteht: einem oberen Zerstäuber, einem unteren Zerstäuber und einem tangentialen Zerstäuber; und/oder
wobei der zweite Zerstäuber ein unterer Zerstäuber ist; und/oder
wobei der zweite Zerstäuber mehrere Lösungsmittel auf das Basisgranulat zerstäubt, um ein mehrschichtiges Basisgranulat auszubilden.

6. Verfahren zum Herstellen der Medien oder der Feed-Zusammensetzung zum Kultivieren von Zellen nach einem der vorhergehenden Ansprüche, wobei der zweite Zerstäuber die mehreren Lösungsmittel nacheinander mit intermittierenden Trocknungsschritten zwischen jedem Zerstäubungsschritt zerstäubt; und/oder
wobei der zweite Zerstäuber die mehreren Lösungsmittel nacheinander zerstäubt, ohne beliebige intermittierende Trocknungsschritte zwischen jedem Sprühschritt.

7. Verfahren nach einem der vorhergehenden Ansprüche, wobei die reaktive Spezies aus der Gruppe ausgewählt ist, die aus einem oder mehreren Spurenelementen, einer oder mehreren reaktiven Aminosäuren, einer oder mehreren reaktiven Aminosäuren, einem oder mehreren Metallsalzen, einer oder mehreren säurelöslichen reaktiven Gruppen, einer oder mehreren alkohollöslichen reaktiven Gruppen und einer oder mehreren pH-Wert-Modifizierungsgruppen besteht.

8. Verfahren nach einem der vorhergehenden Ansprüche, wobei die empfindliche Spezies aus der Gruppe ausgewählt ist, die aus einem oder mehreren Polyaminen, einem oder mehreren Vitaminen, einem oder mehreren Wachstumsfaktoren und einer oder mehreren labilen Aminosäuren besteht; optional wobei das eine oder die mehreren Vitamine aus der Gruppe ausgewählt sind, die aus Vitamin B12, Biotin, Cholin, Folsäure, Niacinamid, Pyridoxin, Riboflavin, Thiamin, Ascorbinsäure und para-Aminobenzoesäure (PABA) besteht.

9. Verfahren zum Herstellen der Medien oder der Feed-Zusammensetzung zum Kultivieren von Zellen nach einem der vorhergehenden Ansprüche, wobei ein Eingas, das in dem Verfahren verwendet wird, eine Temperatur von etwa 20 °C bis 30 °C, optional eine Temperatur von etwa 25 °C aufweist.

10. Verfahren zum Herstellen der Medien oder der Feed-Zusammensetzung zum Kultivieren von Zellen nach einem der vorhergehenden Ansprüche, wobei der Trocknungsschritt bei einer Temperatur von etwa 50 °C bis 60 °C erfolgt.

11. Verfahren zum Herstellen der Medien oder der Feed-Zusammensetzung zum Kultivieren von Zellen nach einem der vorhergehenden Ansprüche, wobei das Basisteilchen oder das Basisgranulat auf einen Feuchtigkeitsgehalt von etwa 0,5 % bis 10 % getrocknet wird.

12. Verfahren zum Herstellen der Medien oder der Feed-Zusammensetzung zum Kultivieren von Zellen nach einem der vorhergehenden Ansprüche, wobei das Lösungsmittel eine reaktive Spezies umfasst.

13. Verfahren zum Herstellen des Mediums oder der Feed-Zusammensetzung zum Kultivieren von Zellen nach einem der vorhergehenden Ansprüche, wobei das Lösungsmittel zusätzlich wenigstens ein Antioxidans umfasst.

14. Verfahren zum Herstellen der Medien oder der Feed-Zusammensetzung zum Kultivieren von Zellen nach einem der vorhergehenden Ansprüche, wobei inerte Komponenten und/oder Quencher wie etwa Antioxidantien mit den empfindlichen Komponenten und/oder den reaktiven Komponenten gemischt werden, oder die inerten Komponenten und/oder Quencher wie etwa Antioxidantien zwischen der reaktiven und der empfindlichen Schicht geschichtet sind.

15. Verfahren zum Herstellen der Medien oder der Feed-Zusammensetzung zum Kultivieren von Zellen nach einem der vorhergehenden Ansprüche, wobei die Komponenten in dem geschichteten Granulat gemäß ihrer chemischen Reaktivität/Eigenschaften; ihrer Anfälligkeit gegenüber thermischen Schäden oder Strahlungsschäden oder photochemischen Schäden oder gemäß ihrer Lösungsmittellöslichkeit segregiert oder gruppiert werden.

## Revendications

1. Procédé de fabrication d'une composition de milieu ou de support nutritif destinée à la culture de cellules, comprenant :
i) le fait de soumettre une poudre sèche à une suspension dans une colonne mobile d'un gaz dans un appareil à lit fluidisé ;
ii) l'introduction d'au moins un solvant à l'aide d'un pulvérisateur dans la poudre sèche de l'étape (i) pour former une particule de base stratifiée ;
iii) le séchage de la particule de base stratifiée ;
chaque solvant de la pluralité de solvants ayant un composant ; chaque solvant ayant des composants différents ; ou chaque solvant ayant un mélange de composants ;
les composants étant séparés en espèces réactives et en espèces sensibles qui sont pulvérisées séparément pour donner un granule stratifié ;
les espèces réactives et les espèces sensibles étant des composants qui interagissent négativement les uns avec les autres.

2. Procédé de fabrication d'une composition de milieu ou de support nutritif destinée à la culture de cellules, comprenant :
i) le fait de soumettre une poudre sèche à une suspension dans une colonne mobile d'un gaz dans un appareil à lit fluidisé ;
ii) l'introduction d'un premier solvant à l'aide d'un premier pulvérisateur sur la poudre sèche de l'étape (i) pour former un granule de base ;
iii) l'introduction d'au moins un second solvant à l'aide d'un second pulvérisateur sur le granule de base de l'étape (ii) pour former un granule de base stratifié ;
iv) le séchage du granule de base stratifié ;
chaque solvant de la pluralité de solvants ayant un composant ; chaque solvant ayant des composants différents ; ou chaque solvant ayant un mélange de composants ;
les composants étant séparés en espèces réactives et en espèces sensibles qui sont pulvérisées séparément pour donner un granule stratifié ;
les espèces réactives et les espèces sensibles étant des composants qui interagissent négativement les uns avec les autres.

3. Procédé de fabrication d'une composition de milieu ou de support nutritif destinée à la culture de cellules selon la revendication 1 ou 2, la poudre sèche étant une poudre de milieu de base, une poudre de milieu complet, un support nutritif de culture cellulaire, un supplément de culture cellulaire, un concentré de milieu ou support nutritif de culture cellulaire ou un mélange d'acides aminés, ou la poudre sèche étant choisie dans le groupe constitué de vitamines, de sels inorganiques en vrac et de sucres.

4. Procédé de fabrication d'une composition de milieu ou de support nutritif destinée à la culture de cellules selon la revendication 1, le premier pulvérisateur étant choisi dans le groupe constitué par : un pulvérisateur supérieur, un pulvérisateur inférieur et un pulvérisateur tangentiel ; et/ou comprenant en outre un second pulvérisateur qui pulvérise une pluralité de solvants sur la particule de base pour former une particule de base multi-stratifiée.

5. Procédé de fabrication d'une composition de milieu ou de support nutritif destinée à la culture de cellules selon la revendication 2 ou 3, le premier ou le second pulvérisateur étant choisi dans le groupe constitué par : un pulvérisateur supérieur, un pulvérisateur inférieur et un pulvérisateur tangentiel ; et/ou
le second pulvérisateur étant un pulvérisateur inférieur ; et/ou
le second pulvérisateur pulvérisant une pluralité de solvants sur le granule de base pour former un granule de base multi-stratifié.

6. Procédé de fabrication d'une composition de milieu ou de support nutritif destinée à la culture de cellules selon l'une quelconque des revendications précédentes, le second pulvérisateur pulvérisant la pluralité de solvants de manière séquentielle, avec des étapes de séchage intermittentes entre chaque étape de pulvérisation ; et/ou
le second pulvérisateur pulvérisant la pluralité de solvants de manière séquentielle, sans aucune étape de séchage intermittente entre chaque étape de pulvérisation.

7. Procédé selon l'une quelconque des revendications précédentes, ladite espèce réactive étant choisie dans le groupe constitué par un ou plusieurs oligo-éléments, un ou plusieurs acides aminés réactifs, un ou plusieurs acides aminés réactifs, un ou plusieurs sels métalliques, un ou plusieurs groupes réactifs solubles dans l'acide, un ou plusieurs groupes réactifs solubles dans l'alcool et un ou plusieurs groupes modificateurs de pH.

8. Procédé selon l'une quelconque des revendications précédentes, ladite espèce sensible étant choisie dans le groupe constitué par une ou plusieurs polyamines, une ou plusieurs vitamines, un ou plusieurs facteurs de croissance et un ou plusieurs acides aminés labiles ; éventuellement la ou les vitamines étant choisies dans le groupe constitué par la vitamine B12, la biotine, la choline, l'acide folique, la niacinamide, la pyridoxine, la riboflavine, la thiamine, l'acide ascorbique et l'acide para-aminobenzoïque (PABA).

9. Procédé de fabrication d'une composition de milieu ou de support nutritif destinée à la culture de cellules selon l'une quelconque des revendications précédentes, un gaz d'admission utilisé dans le procédé étant à une température d'environ 20 °C à 30 °C, éventuellement d'environ 25 °C.

10. Procédé de fabrication d'une composition de milieu ou de support nutritif destinée à la culture de cellules selon l'une quelconque des revendications précédentes, l'étape de séchage étant à une température d'environ 50 °C à 60 °C.

11. Procédé de fabrication d'une composition de milieu ou de support nutritif destinée à la culture de cellules selon l'une quelconque des revendications précédentes, la particule de base ou le granule de base étant séché jusqu'à une teneur en humidité d'environ 0,5 % à 10 %.

12. Procédé de fabrication d'une composition de milieu ou de support nutritif destinée à la culture de cellules selon l'une quelconque des revendications précédentes, le solvant comprenant des espèces réactives.

13. Procédé de fabrication d'une composition de milieu ou de support nutritif destinée à la culture de cellules selon l'une quelconque des revendications précédentes, le solvant comprenant en outre au moins un antioxydant.

14. Procédé de fabrication d'une composition de milieu ou de support nutritif destinée à la culture de cellules selon l'une quelconque des revendications précédentes, des composants inertes et/ou des extincteurs tels que des antioxydants étant mélangés avec les composants sensibles, et/ou, les composants réactifs, ou, les composants inertes et/ou les extincteurs tels que des antioxydants étant stratifiés entre la strate réactive et la strate sensible.

15. Procédé de fabrication d'une composition de milieu ou de support nutritif destinée à la culture de cellules selon l'une quelconque des revendications précédentes, les composants dans le granule stratifié étant séparés ou regroupés en fonction de leur réactivité/propriétés chimiques ; leur susceptibilité aux dommages thermiques ou radiologiques, ou dommages photochimiques, ou selon leur solubilité dans les solvants.
